# EUROPEAN PATENT SPECIFICATION

(11) **EP 4 108 207 B1**
(45) Date of publication and mention of the grant of the patent: **28.02.2024**
(21) Application number: 22151292.4
(22) Date of filing: 13.01.2022
(51) Int. Cl.: A61B 42/40, A61B 42/50, A47G 25/90

(54) **GLOVE DISPENSING APPARATUS**
HANDSCHUHAUSGABEVORRICHTUNG
APPAREIL DE DISTRIBUTION DE GANTS

(30) Priority: 23.06.2021 PL 43823821
(43) Date of publication of application: 28.12.2022
(73) Proprietor: "INNOVA GOOD" SPOLKA Z OGRANICZONA ODPOWIEDZIALNOSCIA, 98-300 Wielun (PL)
(72) Inventor: BARTOS, Jaroslaw, 98-300 Wielun (PL)
(74) Representative: Kancelaria Eupatent.pl Sp. z.o.o

(56) References cited:
- WO-A1-2019/231377
- DE-A1- 19 719 808
- US-A1- 2004 149 788
- US-A1- 2005 155 133
- US-A1- 2007 170 213
- US-A1- 2011 108 587

## Description

### TECHNICAL FIELD

The invention relates to a glove dispensing apparatus. The apparatus is particularly suitable for use in areas where it is important to maintain high standards of hygiene.

### BACKGROUND

There are known various types of dispensers for various articles, including disposable items, such as sanitary products, for example: gloves, masks, safety goggles, or plastic film shoe covers. Dispensing of said articles should be performed while observing rules of hygiene, to limit transfer of microorganisms, e.g., from a user's hand onto articles held in a storage container of the apparatus, in order to maintain proper microbiological purity of the articles to be dispensed. This improves safety of subsequent users.

There are known automatic glove dispensers that are configured to dispense gloves and aid a user in donning (putting on) the gloves in a manner that helps to maintain adequate microbiological safety - limiting the transmission of microorganisms from the products onto the hands of the user of the apparatus, and limiting the transmission of microorganisms from the hands of the user onto the products to be touched by the user.

There are also known glove dispensers that allow a user to only draw gloves, wherein the users put the drawn gloves on hands by their own. Such dispensers do not ensure full microbiological protection - neither for the user nor for the user's surroundings. This is because when the user puts on the gloves, the user may transfer microorganisms (present on user's hands) onto the external surface of the gloves, from where the microorganisms may be further transmitted to the surroundings.

There are also known glove dispensers configured to dispense and aid a user to don gloves in a manner that at least partially limits direct contact of the user with the external surface of the gloves. Those apparatus, also referred to as contactless dispensers, are configured to automatically dispense and spreading a cuff of the glove, which allows donning of the dispensed gloves, thereby the user can put the user's hand into the glove straight after it is dispensed, without the need for manual spreading of the cuff of the glove. This eliminates the risk of the user to touch external surface of the donned glove. Thus, contactless apparatus provide microbiological protection for both the user and the surrounding, which is important in particular in public spaces, e.g., shops, petrol stations, means of mass transportation, railway stations, airports, medical care facilities, hotels, restaurants, etc.

A US patent US7712642 describes a machine for dispensing and donning gloves by using a conveyor belt and a system of cooperating arms, which, by means of a gear system, are slid under the cuff of the glove being dispensed, and then the arms are extended apart to a certain distance relative to each other. The movement of the arms ensures that the cuff of the glove gets turned up and spread so that the user can put on the glove without touching it. This apparatus , however, features a complex construction and requires long waiting time for dispensing and donning the gloves, because each article being drawn by the user is delivered to the glove donning place on bent hook-shaped arms by means of the conveyor belt.

A US patent application US20210015575 describes a glove donning apparatus, including a consisting of a system of grabbers whose working movements enable cuffs of a gloves to stop and to turn up, wherein the donned gloves come with rigid inserts that facilitates the removing of the gloves from the transport assembly of the device.

A US patent US10478001 describes a tool that facilitates donning rubber gloves. The tool has a form of a board with a protruding elongated section to support one portion of the turned-up cuff of a glove. The user can grab with his/her hand the other portion of the glove's cuff thus increasing the glove's opening facilitating donning the glove . However, this solution does not ensure contactless manipulation while donning gloves , which increases the risk of spreading pathogens (via the external surface of the glove) from the user's hand to the environment.

A Polish patent PL233196 describes a system for dispensing protective gloves, consisting of a container with gloves and a hanger for fixing the container, wherein the container takes the form of a bag welded at the top and bottom which is fixed on the hanger; wherein both the top and bottom parts of the bag are fixed to the hanger so that the bag is stretched between the fixing points, and the container has a sealed opening for taking the gloves out, wherein the cuff of a glove is located immediately next to the opening for taking the gloves out.

Documents US 2005/155133 A1, US 2004/149788 A1 and WO 2019/231377 A1 disclose further examples of glove dispensing devices and systems.

### SUMMARY OF THE INVENTION

As follows from the above-mentioned publications, the constructions of glove dispensers undergo constant development in order to increase their working efficiency and ensure contactless operation, including, in particular, limiting the contact of the user's hands with the external surface of the donned gloves, thus reducing the risk of spreading microorganisms through the surface of the gloves to the environment, and at the same time ensuring proper sanitary conditions of the apparatus users.

However, the known glove dispensers still have some disadvantages. One is a complex construction, which may result in apparatus faults, require more frequent servicing, or impose a high price of the apparatus. Moreover, the relatively low storage capacity of known glove storage containers arranged in the dispensing apparatus requires relatively frequent gloves replenishing. Furthermore, longer glove delivery time is due to complicated systems of storage, transport, and manoeuvring of gloves at the point of donning gloves within the apparatus. Moreover, the need to supply with power the contactless gloves dispensing apparatus further limits their usability at facilities without access to electricity, such as car parks, recreational parks, open stadiums, or areas of organized outdoor meetings.

Therefore, there is a need to provide glove dispensing apparatus featuring less complicated construction and limited faultiness. Further, the apparatus shall have a relatively large glove storage capacity and provide short glove delivery time. There is a need to provide a fast and contactless method for dispensing and donning gloves, such as to reduce the risk of spreading various microorganisms, including pathogens transmitted, among others, via human hands.

In one aspect, the invention relates to a glove dispensing apparatus comprising: a glove donning module for providing a glove to be donned by a user; a glove storage module for storing gloves, the glove storage module being arranged next to the glove donning module and comprising a glove container with holders for holding the gloves in the container. The glove container comprises: a wall with an opening for dispensing the gloves therethrough from the glove storage module to the glove donning module, and a glove pressing system for pressing the gloves held in the container against the wall with the opening; wherein the apparatus further comprises an arm swingably mounted near the opening of the wall of the container, said arm being configured to, by means of a swing movement of the arm within the glove donning module: grab a cuff of the glove held in the container through the opening, and spread the grabbed cuff of the glove within the glove donning module.
In the apparatus according to the invention, the function of the glove storage unit is performed by the container located immediately next to the glove donning module wherein the user puts the gloves on. Preferably, the design of the apparatus can be adapted to simultaneously dispense a pair of gloves (for the left and right hands) at the glove donning module. This provides shortening the dispensing route of the gloves from the glove storage module the glove donning module, thus reducing the time needed for the user to draw the gloves. In the apparatus of the invention the presence of means for transporting the gloves is eliminated, because the gloves donning module is separated from the glove storage module (i.e. the container) only by the wall with the opening; through said opening the gloves are directly dispensed to the glove donning module. Thus, the apparatus can have a compact form whilst ensuring automatic dispensing the gloves and simultaneously spreading the cuff(s) of the dispensed glove(s) to allowing the user to put the gloves on user's hands at the donning module, without making skin contact with the outer side of the donned gloves. This is achieved by the arm that is mounted swingably, i.e. so that the arm can swing around an axis, directly at the glove donning module. Therefore, the arm acts as the working element of the apparatus: the arm, by performing a full swing - i.e. moving back and forth between first and second swing position, grabs and opens the cuff of the dispensed glove directly at the glove donning module where the glove can be directly donned; and after the gloves have been drawn by the user, the arm returns to its first swing position (i.e. an initial position).

The apparatus of the invention further features reduced faultiness (inter alia, due to the absence of rotary shafts), requires less frequent servicing, as well as lowered overall maintenance and operating costs. What is more, the apparatus can exist in a purely mechanical version, without the need for a power supply.

Preferably, the arm is swingably mounted to a body of the apparatus for the swing movement about an axis which is substantially parallel to the wall with the opening of the container, wherein said axis is arranged in front of said wall, next to the glove donning module.
This allows the arm to be installed in front of the glove storage module, thus providing further reduction in the size of the apparatus and a shorter path of the swing movement of the arm, while maintaining a simple design of the apparatus. This is due to the implementation of the arm, whose axis is arranged essentially parallel to the wall of the container, thus the arm can swing out of its first swing position in the direction opposite to said wall, falling at the same time onto the cuff of the glove, thereby the arm engages with the glove - directly at the glove donning module, so that the cuff of the glove, guided by the falling arm, becomes spread apart and the user is able to contactlessly put on the glove, at the glove donning module.

Preferably, the arm comprises: a grabbing section arranged on one side of said axis, movable along a path of movement arranged within the glove donning module, for grabbing and spreading the cuff of the glove held in the container, and a manoeuvring section arranged on the other side of the axis for manoeuvring the arm. The path of movement of the grabbing section is arranged so that the swing movement of the arm engages the movement of the grabbing section along the path of the movement involving the apparatus to transit: from a standby mode of the apparatus in which the grabbing section is not engaged with the cuff the glove held in the glove storage module, into a working mode of the apparatus in which the grabbing section grabs and spreads said cuff of said glove, within the donning module, as a result of the movement of the grabbing section form its first swing position, defining a first end of the path of movement, into its second swing position, defining a second end of the path of movement.
Such a design ensures more hygienic conditions of use of the apparatus. The swing movement of the grabbing section, which comes in direct contact with the glove, is accomplished using the manoeuvring section that protrudes beyond the glove donning module - onto the other side of the axis. The movement of the manoeuvring section of the arm engages the swing movement of the grabbing section of the arm so that the grabbing section swings out of its first swing position (the arm falls down in a swing motion) simultaneously gripping and spreading apart the cuff of the glove directly from the storage module, at the glove donning module where the user can don the grabbed and spread glove. For example, the manoeuvring section of the arm can be provided with a hand-operated lever or a button protruding from the apparatus body so that the user, by moving the lever or pressing the button, causes movement of the grabbing section and thereby dispensing the glove at the glove donning module.

Preferably, the apparatus further comprising a movable pedal engaged movably with the arm so that a movement of the pedal causes the swing movement of the arm.
The pedal further improves the sanitary conditions of the apparatus usage. The pedal can, for example, be adapted to be operated with a foot, so that the user, by pressing the pedal with user's foot, causes the apparatus to transit from the standby mode to the working mode essentially contactlessly (without using user's hands).

Preferably, the pedal is engaged movably with the manoeuvring section of the arm.
This provides the additional physical separation of the grabbing section from the other elements of the apparatus, which provides reduced risk of transferring microorganisms, e.g., from the apparatus body onto the user's hands or *vice versa.*

Preferably, the pedal is engaged movably with the arm via a connecting member for transmitting the movement of the pedal to the manoeuvring section of the arm.
This provides the additional physical separation of the grabbing section from the other elements of the apparatus, which provides further reduction of the risk of transferring microorganisms.

Preferably, the connecting member constitutes a rigid bar articulatedly jointed to the pedal and to the arm.
This increases the rigidity of the apparatus construction as well as the efficiency of the apparatus operation; further, this allows the dimensions of the machine to be adapted to the height of the target users - smaller machines can be provided with shorter connecting members, and larger machines can be provided with longer connecting members.

Preferably, the pedal is swingably mounted in the apparatus.
This provides further increased rigidity of the apparatus construction as well as reliability of the apparatus operation. The apparatus with the pedal comprising configured to swingably move around an axis, can exist in a version without power supply.

Preferably, the apparatus further comprising a spring means for connecting the pedal with the apparatus body to springily tension the pedal to cause transition of the apparatus from the working mode into the standby mode after the movement of the pedal.
The advantage of the above is that the apparatus can automatically by means of spring action) return from the working mode to the standby mode.

Preferably, the pedal is mounted at a base of the apparatus.
This enables the apparatus to be operated solely by using the user's foot (e.g. wearing a shoe), thus limiting the contact of the user's hand with the apparatus body. This ensures high hygiene standards for the apparatus users (the user does not touch the apparatus directly with hands), thereby, spreading of various microorganisms, including pathogens is limited; further this reduces the frequency of required sanitization (washing) of the apparatus body.

Preferably, the arm is mounted above the pedal, preferably from 70 to 120 cm above the base of the apparatus.
This enables adjusting the place of the glove donning module to the height of the users, while at the same time implementing the pedal of the apparatus at the height of their feet.

Preferably, the container of the glove storage module comprises a door for accessing the interior of the container and replenishing the container with the gloves.
This facilitates periodic replenishment of the glove storage module and provides physical separation of the gloves stored in the apparatus storage module from the surroundings.

Preferably, the glove pressing system includes a spring means ended with a pressure plate for springily pressing the gloves in the container against the wall with the opening.
This allows for storing a larger number of gloves in the container, preferably up to 1000 gloves for the right hand, for example coming in ten stacks of 100 gloves each, and preferably up to 1 000 gloves for the left hand, for example coming in ten stacks of 100 gloves each, i.e., up to 2 000 gloves in total coming in twenty stacks, ensuring an appropriate pressing of the gloves against the wall in the opening of the container and efficient dispensing of the gloves at the glove donning module. Moreover, the container may be configured to hold a different maximum number of gloves at a time (more or less than 2 000 gloves) - depending on the parameters of the spring means for pressing the gloves stacked in the container, and depending on the desired dimensions of the container.

Preferably, the pressing plate comprises a guide for guiding the pressing plate, the guide wherein articulatedly joints the pressing plate with the door of the container.
This allows for wide availability of spare parts, and thus lower costs associated with maintenance of the apparatus.
Further, This ensures that the gloves are uniformly pressed by the pressing plate, in the container, and that the gloves are stabilized, while allowing quick dispensing of the gloves from the container.

Preferably, the container of the glove storage module further comprises a tray arranged in a lower section of the container for collection of waste generated after dispensing and donning of gloves.
This allows for keeping tidiness near the apparatus.

Further aspects and features of the present invention are described in following description of the drawings.

### BRIEF DESCRIPTION OF DRAWINGS

Aspects and features of the present invention will become apparent by describing, in detail, an exemplary embodiment of the present invention with reference to the attached drawings, in which:
Fig. 1A-1B show a cross-sectional view of a glove dispensing apparatus, in a standby mode (Fig. 1A) and in a working mode (Fig. 1B);
Fig. 1C-1D show two perspective views of the apparatus;
Fig. 2 schematically shows the principle of operating the apparatus;
Fig. 3 shows the glove donning module and an outline of a path of movement of an arm, in a cross-sectional view through the apparatus;
Fig. 4 shows an inside of a container of the apparatus, with a door open;
Fig. 5 shows an enlarged view of: the glove donning module (Fig. 5A), the arm of the apparatus (Fig. 5B), the apparatus pedal, a connecting member (Fig. 5C), and the tray for wastes arranged in a lower section of the container (Fig. 5D);
Fig. 6 shows a glove to be dispensed by the apparatus, with the visible cuff section of the glove.

### DETAILED DESCRIPTION

Reference will now be made to embodiments, examples of which are illustrated in the accompanying drawings. Aspects and features of the embodiments will be described with reference to the accompanying drawings. In the drawings, like reference numerals denote like elements. The present invention, however, may be embodied in various different forms and should not be construed as being limited only to the illustrated embodiments. Rather, these embodiments are provided as examples so that this disclosure will be thorough and complete, and will fully convey the aspects and features of the present invention to those skilled in the art. It shall be understood that not all of the features shown in the embodiments are essential and the scope of the protection is defined not by means of literally shown embodiments, but by the features provided in the claims.

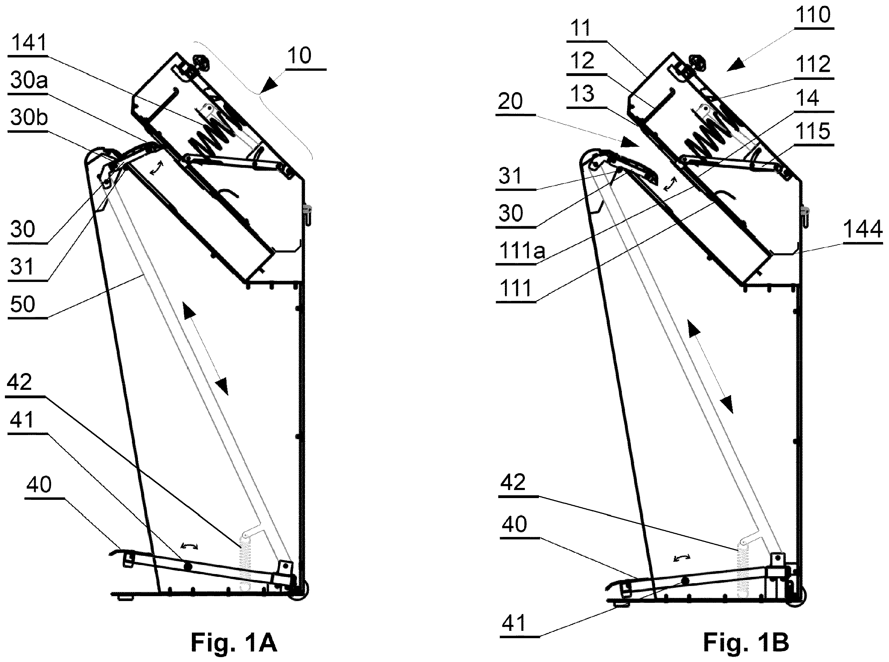

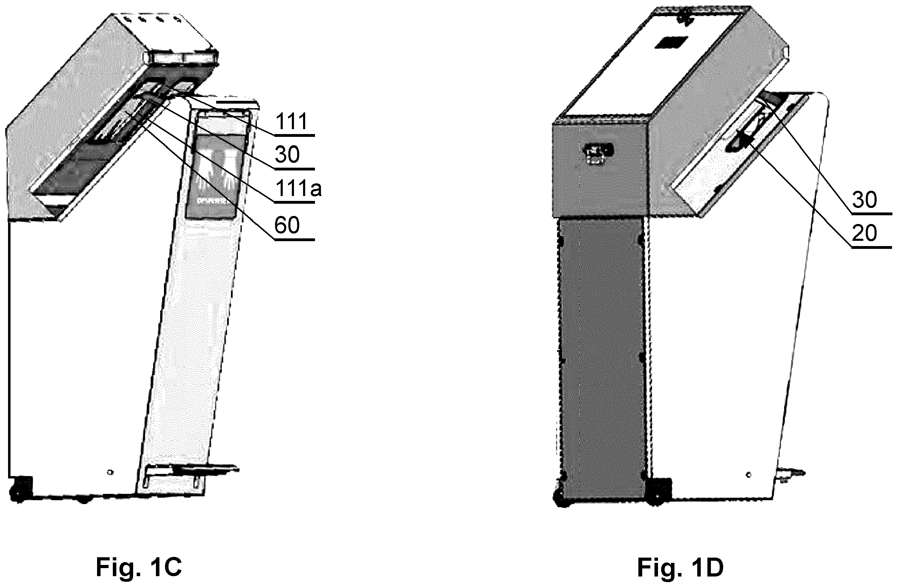

Fig. 1 presents an embodiment of the glove dispensing apparatus. Preferably, the apparatus takes a form similar to that of a geometric prism with a quadrilateral base, e.g., square or rectangular base, with a glove donning module 20, e.g., in the shape of a recess in a body of the apparatus, for inserting the user's hands. The recces preferably can be arranged at the height of the user's waist, to ensure convenient donning of gloves dispensed by the apparatus, i.e., gloves with automatically spread cuffs that the user directly puts on his/her hands (or one hand) at the glove donning module 20.

The apparatus comprises a glove storage module 10 arranged directly next to the donning module 20. The glove storage module 10 comprises a container 11 for storing gloves, preferably in the form of a chamber having a wall 111 arranged as a stationary wall, and provided with an opening 111a performed therein; the wall 111 separates the interior of the container 11 from the glove donning module 20. The container 11 further comprises a second wall arranged as a movable wall facing the wall 111 with the opening 111a. The movable wall is in the form of a pressing plate 142 constituting the moving element of a gloves pressing system 14. The pressing plate 142 is configured for pressing the gloves in the container 11 against the wall 111 with an opening 111a, which facilitates storing the gloves 60 each arranged flat one glove on top of the other, thereby forming a stack 70 of gloves 60, in the container 11. The opening 111a of the wall 111 constitutes an outlet for dispensing the gloves stored in the container 11, directly to the glove donning module 20.

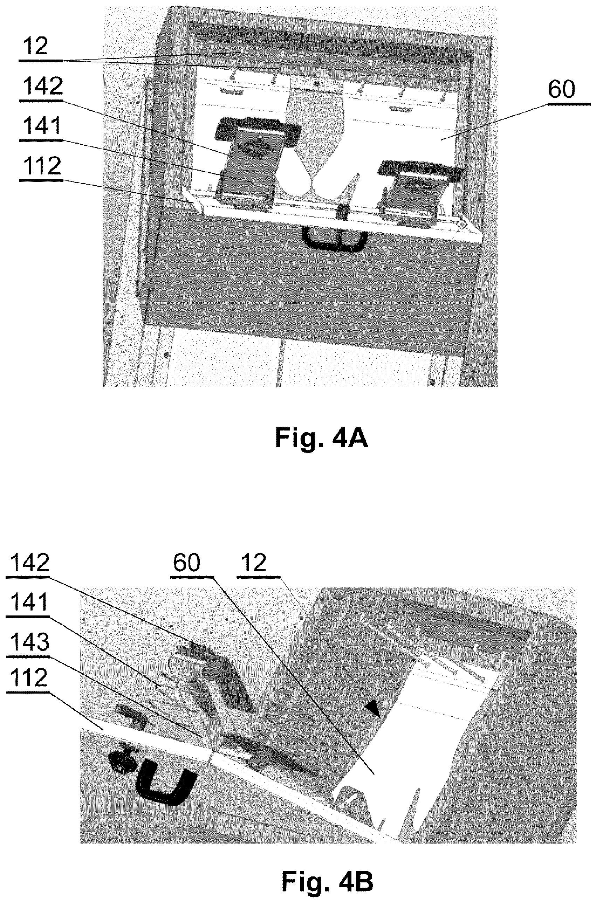

The glove storage module 10 comprises the pressing system 14 for pressing the gloves stored in the container 11 against the wall 111 with the opening 111a, as described above. The pressing system 14 comprises said pressing plate 142, taking the form of a movable wall of the container 11 installed opposite the wall 111 that is stationary, as the container further comprises a door 112 of the container 11 installed as an element of the apparatus body. The pressure plate 142 of the pressing system 14 is installed springily, inside the container 11, and it is connected via spring means 141 and via a guide 143 to the door 112 of the container 11, as schematically shown in Fig. 4A and 4B - with the door 112 of the container 11 opened. This construction facilitates replenishing the container 11 with new gloves 60.

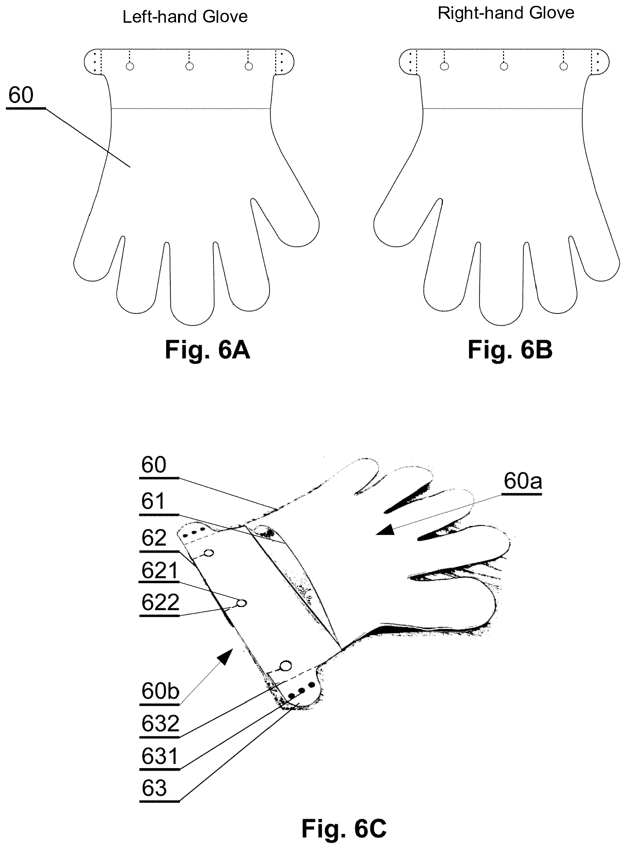

Above the opening 111a, the container 11 has holders 12 for holding the gloves 60 stored in the container. The holders protrude from the wall 111 into the interior of the container 11. The holders 12 may take the form of elongated hooks, for example two, three, or more than three hooks, on which the gloves 60 are hung in the container 11; the holders 12 may be arranged in a pattern of line. For example, as shown in Fig. 4A and 4B, in one embodiment, the apparatus may be provided with holders 12 for right and left gloves (Fig. 6A and 6B) hold in the common container 11, for simultaneous dispensing a complete set of gloves - to be donned as a pair, for the right and left hand.

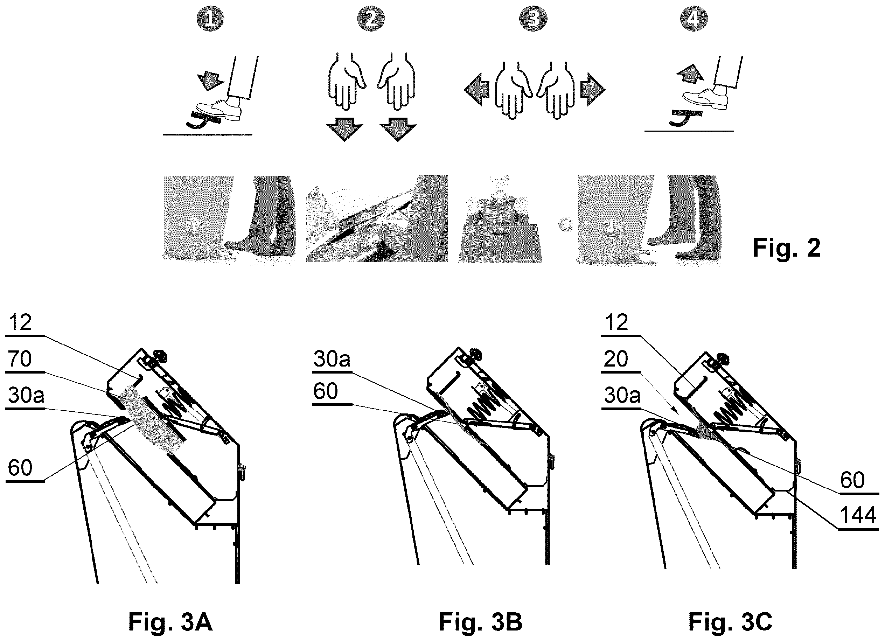

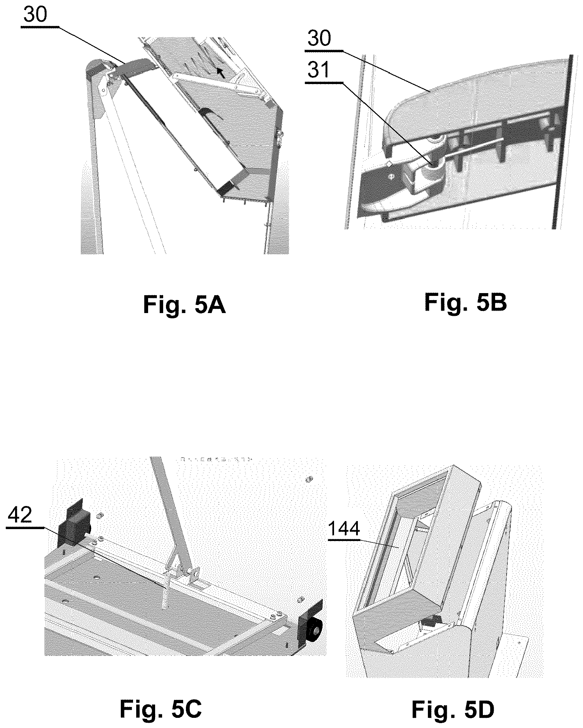

The design of the gloves 60 shown in Fig. 6, is suitable for hanging the gloves on holders 12 in the container 11, including hanging singular gloves (Fig. 3B) or hanging gloves arranged in stacks 70 (Fig. 3A). Namely: each glove 60 has a short cuff 61 on its grip side 60a and a long cuff 62 on its rear side 60b provided with openings 621 for holders 12 on the rear side 60b of the glove 60. Preferably, the openings 621 are connected to the edge of the long cuff 62 by a tear line 622 in the form of short cuts formed in the material of the glove; each tear line extends from the edge of each opening 621 to the edge of the long cuff 62. The tear lines facilitate tearing the glove 60 off the holders 12 during the donning of gloves. Furthermore, the long cuff 62 of each glove may have laterally projecting segments 63, for example two segments 63, each in the form of a small flap. The segments 63, can comprise connecting spots 631 for example in the form of spot welds 631, for example two or three spot welds 631. The connection spots 631 connect gloves 60 arranged in the single stack 70, one on top of the other. The connection spots 631 ensure a stable arrangement of gloves in the stack 70, which facilitates replenishing of the container 11 with new stacks 70 of gloves 60, and further facilitates dispensing the gloves 60 - when arranged in the stacks 70, as well as allows storing more gloves in the container 11, thereby increasing the apparatus capacity - each stack 70 of gloves 60, where the individual gloves 60 are connected by welds 631, may comprise preferably up to one hundred gloves (on common holders 12), and the container 11 may store, for example, ten stacks 70 of gloves 60, which can be faultlessly dispensed by the apparatus, and thereby comfortably donned. Moreover, each segment 63 may be connected with the cuff of the glove via a tear line 632. Due to that, drawing by the user the donned gloves, involving tearing apart of the tear lines 622, 632, is carried out waste less: the segments 63 of donned gloves 60, after tearing, remain inside the container 11 because they are connected to the segments 63 of the gloves in the stack 70 held in the container 11, on the holders 12. This is because the segments 63 of gloves in stacks 70 are connected together by the connection spots 631, wherein each glove 60 (right hand glove, left hand glove) may have two segments 63 extending from the glove 60 in opposite directions. This design, in case of donning the last glove 60 from the stack 70, enables the segments 60 to gravitationally fall into the waste tray 144 (Fig. 5D) located at the bottom of the container 11. From the tray 144, the waste segments 63 of the donned gloves 60 may be periodically removed, e.g., when replenishing the container 11 with new gloves. This allows to keep the area around the machine tidy and to reduce the amount of material used for gloves to a minimum. An advantage of the design of gloves 60 with segments 63 extending laterally form each glove 60 is that the pressing system 14 ensures proper pressing of the gloves 60 in the stacks 70 stored in the container 11, while omitting (not pressing) the segments 63. Namely: the surface area of the pressing plates 142 does not cover the segments 63 of gloves 60 held on the common hangers 12 in the container 11. Therefore, as the number of gloves 60 in the stacks 70 decreases (being the result of dinning the gloves dispensed from the container 11), the force with which the gloves 60 are pressed against the wall 111 with the opening 111a (exerted by the pressing system 14) remains relatively constant as the remained segments 63 from the donned gloves protruding out of the stack 70 are not pressed by the glove pressing plates.

After all the gloves from the container 11 have been dispensed, i.e., after one full load has been consumed - for example, up to 1,000 right hand gloves, i.e., for example, coming as ten stacks 70 of right hand gloves 60 - hold on common holders 12, and, for example, up to 1,000 left hand gloves, i.e., for example, coming as ten stacks 70 of left hand gloves 60 hold on common holders 12 (i.e., up to 2,000 gloves in total), coming as total of twenty stacks 70 of gloves 60 - twice as many segments 63 may be collected in the waste tray 144 (Fig. 5D), i.e., for example, up to forty stacks of segments 63 joined by welds 631; waste segments 63 left from the donned gloves 60 may be removed from the tray 144 before each replenishment of the container 11 with new gloves 60 (Fig. 6A and 6B). The size of the gloves 60 and the length of each of the cuffs 61, 62 of the gloves 60 are adapted to the height of the holders 12 and to the size of the opening 111a in the immovable wall 111 of the container 11. As schematically shown in Fig. 3A, the central part and the short cuff 61 of a glove are exposed in the opening 111a of the wall 111 of the container 11, the edges of the glove rest against the wall 111 on the inner side of the container 11, and the long cuffs 62 of the gloves are held on the holders12.

The apparatus includes a arm 30 mounted swingably for grabbing, through the opening 111a, the short cuff 61 of a glove held on the holders 12, in the container 11, and for spreading the cuffs 61, 62 of said glove 60 as a result of a swing movement of the arm 30 at the glove donning module 20. In one of the embodiments, the apparatus can comprise one arm 30, and in another embodiment the apparatus can comprise two arms 30 for simultaneously dispensing and donning a pair of gloves - right and left, thereby, performing the same operations in parallel by two arms 30.

As shown in Fig. 1, the arm 30 is swingably mounted to the body of the apparatus for a swing movement about an axis 31. The axis 31 extends substantially parallel to the wall 111 of the container 11, in front of the wall 111 of the container 11, next to the glove donning module 20. On one side of the axis 31, the arm has a grabbing section 30a (shown in Fig. 5A and 5B), and on the other side of the rotation axis 31, the arm 30 has a manoeuvring section 30b for manoeuvring the arm 30 to cause the grabbing section to grab and spread the cuffs 61, 62 of gloves 60. In the embodiment providing simultaneous dispense of gloves pair - right and left glove, the apparatus, may include two parallel grabbing sections 30a of the arm 30 and one or two manoeuvring sections 30b, for performing the same movements in parallel. A path of movement of each grabbing section 30a is adapted in such a way that, as a result of the swing movement of the arm 30 away from its position of balance (i.e. a first swing position), the grabbing section 30a of the arm 30 moves along the path of movement, thereby, the apparatus transits from the standby mode (Fig. 3A, 3B) - in which the grabbing section 30a is not engaged with the glove's cuff, into the working mode of the apparatus (automatic spreading of glove cuffs), in which the grabbing section 30a engages and then spreads the cuff of the glove 60, a result of swing movement of the grabbing section 30a forth (in one direction), between first and second swing position, at the glove donning module 20. This provides exposing the glove's inlet to the user (Fig. 3C), and thereby dispensing the glove, at the glove donning module 20. After donning the gloves, as a result of swing movement of the grabbing section 30a back (in the opposite direction), between second and first swing position, the grabbing section 30a returns to its first swing position, thereby, the apparatus returns from the working mode (Fig. 3C) to the standby mode (Fig. 3A), and so the grabbing section 30a performs a full swing - back and forth between the first and second swing position, at the glove donning module 20. This working principle of the apparatus is achieved because the short cuff 61 of the glove 60 is exposed through the opening 111a of the wall 111 of the container 11 directly towards the glove donning module 20, and the grabbing section 30a moves along the path of movement at the glove donning module 20, wherein the grabbing section 30a in course of its movement engages the edge of the exposed short cuff 61 of the glove thereby moving the cuff 61 away from the container 11, through the opening 111a, directly at the glove collection point 20, whist leaving the second (long) cuff 62 of the glove intact.

The grabbing section 30a may be curved and may have a pointy end to effectively grab the short cuff 61 of the glove 60 from the container 11. The apparatus may further comprise a pedal 40 swingably mounted in the apparatus so that the pedal swings around an axis 41 which basically parallel to the axis 31 of the arm 30. The pedal 40 can be connected with the arm 30 via a connecting member 50 for transmitting a movement of the pedal 40 to the arm 30. The connecting member can be articulatedly joined to the arm 30, at one end, and articulatedly jointed to the pedal 40, at the other end. This enables the user to activate the machine - from the standby mode to the working mode - by pressing the pedal 40 with the user's foot, i.e. without using his/her hands (contactlessly). Preferably, the axes 31 and 41 are arranged along shafts installed in the apparatus body. The pedal 40 may be connected to the apparatus body via spring means 42 (Fig. 5C), e.g., a spring or an elastic strap to sparingly tension/maintain the pedal 40 in its initial position (Fig. 1A) i.e. at the apparatus standby mode.

Furthermore, the apparatus may comprise wheels, installed at its base, for example, two wheels mounted to a common axle. The wheels facilitate transporting of the machine over the ground.

Fig. 2 schematically shows user's manual of the apparatus. Pressing the pedal with the user's foot induces the pedal 40 movement (illustration 1), the pressing force is transmitted via the connecting member 50 to the arm 30. The arm 30 upon the force acing thereon swings and thereby grabs and spreads the cuff 61 of the glove 60 at the glove donning module 20 (illustration 2) - at this stage the pedal remains pressed by the user. The user inserts his/her hands directly into the gloves 60 at glove donning module 20. After donning the gloves, the user moves his/her gloved hands apart (two hands in gloves are moved laterally, in the opposite directions) thereby tearing the gloves 60 at tear lines 622, 632 off, and removing his/her hands from the glove donning module 20 (illustration 3). Next, the user releases the pedal (illustration 4), upon the release the pedal returns to its initial position by means of the spring means 42, thereby the arm 30 perform one complete swing (movement back and forth between first and second swing position), and the apparatus returns from the working mode to the standby mode.

The developed construction and operating mode of the apparatus provides a larger capacity of the glove container 11. As a result, a large number of gloves in stacks (packets) can be loaded into the container 11 of the machine at once. Further, due to the adapted pressing system 14, the gloves stored in the container 11 do not move, fold, or crease during their automatic dispensing, so gloves stored in the machine may be used up entirely in accordance with their intended purpose, without generating any excess waste. Moreover, the apparatus can operate purely mechanically, without the need for power supply. This is because the swing movement of the arm 30, which can be caused directly by the user, covers the entire process of dispensing the gloves to the user, i.e., the full swing of the arm. Due to that, in the embodiment of the apparatus without the power supply, operation of the apparatus does not negatively affect the natural environment and the apparatus can be used in various areas without any restrictions, e.g., at shops, petrol stations, car parks, outdoor meeting places, etc.

## Claims

1. A glove dispensing apparatus comprising:
- a glove donning module (20) for providing a glove to be donned by a user;
- a glove storage module (10) for storing gloves, the glove storage module (10) being arranged next to the glove donning module and comprising a glove container (11) with holders (12) for holding the gloves in the container (11),
- wherein the glove container (11) comprises:
- a wall (111) with an opening (111a) for dispensing the gloves therethrough from the glove storage module (10) to the glove donning module (20), and
- a glove pressing system (14) for pressing the gloves held in the container (11) against the wall (111) with the opening (111a),
**characterized in that**:
- the apparatus further comprises an arm (30) swingably mounted near the opening (111a) of the wall (111) of the container (11), said arm (30) being configured to, by means of a swing movement of the arm (30) within the glove donning module (20):
- grab a cuff of the glove held in the container (11) through the opening (111a), and
- spread the grabbed cuff of the glove within the glove donning module (20).

2. The apparatus according to claim 1, wherein the arm (30) is swingably mounted to a body of the apparatus for the swing movement about an axis (31) which is substantially parallel to the wall (111) with the opening (111a) of the container (11), wherein said axis is arranged in front of said wall (111), next to the glove donning module (20).

3. The apparatus according to claim 2, wherein the arm (30) comprises:
- a grabbing section (30a) arranged on one side of said axis (31), movable along a path of movement arranged within the glove donning module (20), for grabbing and spreading the cuff of the glove held in the container (11), and
- a manoeuvring section (30b) arranged on the other side of the axis (31) for manoeuvring the arm (30),
- wherein the path of movement of the grabbing section (30a) is arranged so that the swing movement of the arm (30) engages the movement of the grabbing section (30a) along the path of the movement involving the apparatus to transit:
- from a standby mode of the apparatus in which the grabbing section (30a) is not engaged with the cuff the glove held in the glove storage module (10),
- into a working mode of the apparatus in which the grabbing section (30a) grabs and spreads said cuff of said glove, within the donning module (20), as a result of the movement of the grabbing section (30a) form its first swing position, defining a first end of the path of movement, into its second swing position (30a), defining a second end of the path of movement.

4. The apparatus according to any of the above claims, further comprising a movable pedal (40) engaged movably with the arm (30) so that a movement of the pedal (40) causes the swing movement of the arm (30).

5. The apparatus according to claim 4, wherein the pedal (40) is engaged movably with the manoeuvring section (30b) of the arm (30).

6. The apparatus according to claim 4 or 5, wherein the pedal (40) is engaged movably with the arm (30) via a connecting member (50) for transmitting (40) the movement of the pedal (40) to the manoeuvring section (30b) of the arm (30).

7. The apparatus according to claim 6, wherein the connecting member (50) constitutes a rigid bar articulatedly jointed to the pedal (40) and to the arm (30).

8. The apparatus according to any of claims 4 to 6, wherein the pedal (40) is swingably mounted in the apparatus.

9. The apparatus according to any of claims 4 to 8, further comprising a spring means (42) for connecting the pedal (40) with the apparatus body to springily tension the pedal (40) to cause transition of the apparatus from the working mode into the standby mode after the movement of the pedal (40).

10. The apparatus according to any of claims 4 to 9, wherein the pedal (40) is mounted at a base of the apparatus.

11. The apparatus according to claim 10, wherein the arm (30) is mounted above the pedal (40), preferably from 70 to 120 cm above the base of the apparatus.

12. The apparatus according to any of the above claims, wherein the container (11) of the glove storage module (10) comprises a door (112) for accessing the interior of the container (11) and replenishing the container (11) with the gloves.

13. The apparatus according to any of the above claims, wherein the glove pressing system (14) includes a spring means (141) ended with a pressure plate (142) for springily pressing the gloves in the container (11) against the wall (111) with the opening (111a).

14. The apparatus according to claim 13, wherein the pressing plate (142) comprises a guide (143) for guiding the pressing plate (142), the guide (143) wherein articulatedly joints the pressing plate (142) with the door (112) of the container (11).

15. The apparatus according to any of the above claims, wherein the container (11) of the glove storage module (10) further comprises a tray (144) arranged in a lower section of the container (11) for collection of waste generated after dispensing and donning of gloves.

## Patentansprüche

1. Handschuhausgabevorrichtung, umfassend:
- ein Handschuhanziehmodul (20) zum Bereitstellen eines Handschuhs, der durch einen Benutzer anzuziehen ist;
- ein Handschuhaufbewahrungsmodul (10) zum Aufbewahren von Handschuhen, wobei das Handschuhaufbewahrungsmodul (10) neben dem Handschuhanziehmodul angeordnet ist und einen Handschuhbehälter (11) mit Halterungen (12) zum Halten der Handschuhe in dem Behälter (11) umfasst,
- wobei der Handschuhbehälter (11) Folgendes umfasst:
- eine Wand (111) mit einer Öffnung (111a) zum Ausgeben der Handschuhe dort hindurch von dem Handschuhaufbewahrungsmodul (10) zu dem Handschuhanziehmodul (20), und
- ein Handschuhdrücksystem (14) zum Drücken der Handschuhe, die in dem Behälter (11) gehalten werden, gegen die Wand (111) mit der Öffnung (111a),
**dadurch gekennzeichnet, dass**:
- die Vorrichtung ferner einen Arm (30) umfasst, der schwenkbar nahe der Öffnung (111a) der Wand (111) des Behälters (11) montiert ist, wobei der Arm (30) konfiguriert ist, um mittels einer Schwenkbewegung des Arms (30) innerhalb des Handschuhanziehmoduls (20):
- eine Manschette des Handschuhs, der in dem Behälter (11) gehalten wird, durch die Öffnung (111a) zu greifen, und
- die ergriffene Manschette des Handschuhs innerhalb des Handschuhanziehmoduls (20) zu spreizen.

2. Vorrichtung nach Anspruch 1, wobei der Arm (30) schwenkbar an einem Körper der Vorrichtung für die Schwenkbewegung um eine Achse (31), die im Wesentlichen parallel zu der Wand (111) mit der Öffnung (111a) des Behälters (11) ist, montiert ist, wobei die Achse vor der Wand (111) neben dem Handschuhanziehmodul (20) angeordnet ist.

3. Vorrichtung nach Anspruch 2, wobei der Arm (30) Folgendes umfasst:
- einen Greifabschnitt (30a), der auf einer Seite der Achse (31) angeordnet ist, bewegbar entlang eines Bewegungsweges, der innerhalb des Handschuhanziehmoduls (20) angeordnet ist, um die Manschette des Handschuhs, der in dem Behälter (11) gehalten wird, zu greifen und zu spreizen, und
- einen Manövrierabschnitt (30b), der auf der anderen Seite der Achse (31) angeordnet ist, um den Arm (30) zu manövrieren,
- wobei der Bewegungsweg des Greifabschnittes (30a) angeordnet ist, sodass die Schwenkbewegung des Arms (30) die Bewegung des Greifabschnittes (30a) entlang des Bewegungsweges in Eingriff nimmt, involvierend, dass die Vorrichtung übergeht:
- aus einem Standby-Modus der Vorrichtung, in dem der Greifabschnitt (30a) nicht mit der Manschette des Handschuhs, der in dem Handschuhaufbewahrungsmodul (10) gehalten wird, in Eingriff ist,
- in einen Arbeitsmodus der Vorrichtung, in dem der Greifabschnitt (30a) die Manschette des Handschuhs innerhalb des Anziehmoduls (20) greift und spreizt, als Ergebnis der Bewegung des Greifabschnittes (30a) aus seiner ersten Schwenkposition, die ein erstes Ende des Bewegungsweges definiert, in seine zweite Schwenkposition (30a), die ein zweites Ende des Bewegungsweges definiert.

4. Vorrichtung nach einem der obigen Ansprüche, ferner umfassend ein bewegbares Pedal (40), das bewegbar mit dem Arm (30) in Eingriff ist, sodass eine Bewegung des Pedals (40) die Schwenkbewegung des Arms (30) bewirkt.

5. Vorrichtung nach Anspruch 4, wobei das Pedal (40) bewegbar mit dem Manövrierabschnitt (30b) des Arms (30) in Eingriff ist.

6. Vorrichtung nach Anspruch 4 oder 5, wobei das Pedal (40) bewegbar mit dem Arm (30) über ein Verbindungselement (50) in Eingriff ist, um die Bewegung des Pedals (40) auf den Manövrierabschnitt (30b) des Arms (30) zu übertragen (40).

7. Vorrichtung nach Anspruch 6, wobei das Verbindungselement (50) eine starre Stange darstellt, die gelenkig mit dem Pedal (40) und mit dem Arm (30) verknüpft ist.

8. Vorrichtung nach einem der Ansprüche 4 bis 6, wobei das Pedal (40) schwenkbar in der Vorrichtung montiert ist.

9. Vorrichtung nach einem der Ansprüche 4 bis 8, ferner umfassend ein Federmittel (42) zum Verbinden des Pedals (40) mit dem Vorrichtungskörper, um das Pedal (40) federnd zu spannen, um Übergang der Vorrichtung von dem Arbeitsmodus in den Standby-Modus nach der Bewegung des Pedals (40) zu bewirken.

10. Vorrichtung nach einem der Ansprüche 4 bis 9, wobei das Pedal (40) an einer Basis der Vorrichtung montiert ist.

11. Vorrichtung nach Anspruch 10, wobei der Arm (30) über dem Pedal (40) montiert ist, bevorzugt 70 bis 120 cm über der Basis der Vorrichtung.

12. Vorrichtung nach einem der obigen Ansprüche, wobei der Behälter (11) des Handschuhaufbewahrungsmoduls (10) eine Tür (112) zum Zugreifen auf das Innere des Behälters (11) und Auffüllen des Behälters (11) mit den Handschuhen umfasst.

13. Vorrichtung nach einem der obigen Ansprüche, wobei das Handschuhdrücksystem (14) ein Federmittel (141) beinhaltet, das mit einer Druckplatte (142) endet, um die Handschuhe in dem Behälter (11) federnd gegen die Wand (111) mit der Öffnung (111a) zu drücken.

14. Vorrichtung nach Anspruch 13, wobei die Druckplatte (142) eine Führung (143) zum Führen der Druckplatte (142) umfasst, wobei die Führung (143) die Druckplatte (142) gelenkig mit der Tür (112) des Behälters (11) verknüpft.

15. Vorrichtung nach einem der obigen Ansprüche, wobei der Behälter (11) des Handschuhaufbewahrungsmoduls (10) ferner eine Schale (144) umfasst, die in einem unteren Abschnitt des Behälters (11) zur Sammlung von Abfall, der nach dem Ausgeben und Anziehen von Handschuhen erzeugt wird, angeordnet ist.

## Revendications

1. Appareil de distribution de gants comprenant :
- un module d'enfilage (20) de gants destiné à fournir un gant devant être enfilé par un utilisateur ;
- un module de stockage (10) de gants destiné à stocker des gants, le module de stockage (10) de gants étant agencé à côté du module d'enfilage de gants et comprenant un conteneur à gants (11) avec des supports (12) destinés à supporter les gants dans le conteneur (11),
- ledit conteneur à gants (11) comprenant :
- une paroi (111) comportant une ouverture (111a) en vue de la distribution des gants à travers celle-ci à partir du module de stockage (10) de gants jusqu'au module d'enfilage (20) de gants, et
- un système de pressage (14) de gants pour presser les gants supportés dans le conteneur (11) contre la paroi (111) avec l'ouverture (111a), **caractérisé en ce que** :
- l'appareil comprend en outre un bras (30) monté oscillant à proximité de l'ouverture (111a) de la paroi (111) du conteneur (11), ledit bras (30) étant conçu pour, au moyen d'un mouvement d'oscillation du bras (30) au sein du module d'enfilage (20) de gants :
- saisir un revers du gant supporté dans le conteneur (11) à travers l'ouverture (111a), et
- déployer le revers saisi du gant à l'intérieur du module d'enfilage (20) de gants.

2. Appareil selon la revendication 1, ledit bras (30) étant monté oscillant sur un corps de l'appareil pour le mouvement d'oscillation autour d'un axe (31) qui est sensiblement parallèle à la paroi (111) avec l'ouverture (111a) du conteneur (11), ledit axe étant agencé devant ladite paroi (111), à côté du module d'enfilage (20) de gants.

3. Appareil selon la revendication 2, ledit bras (30) comprenant :
- une section de préhension (30a) agencée sur un côté dudit axe (31), mobile le long d'un trajet de mouvement agencé à l'intérieur du module d'enfilage (20) de gants, pour saisir et déployer le revers du gant supporté dans le conteneur (11), et
- une section de manoeuvre (30b) agencée de l'autre côté de l'axe (31) destinée à la manoeuvre du bras (30),
- ledit trajet de mouvement de la section de préhension (30a) étant agencée afin que le mouvement d'oscillation du bras (30) engage le mouvement de la section de préhension (30a) le long du trajet du mouvement impliquant l'appareil pour effectuer une transition :
- d'un mode veille de l'appareil dans lequel la section de préhension (30a) n'est pas en prise avec le revers du gant maintenu dans le module de stockage (10) de gants,
- à un mode de fonctionnement de l'appareil dans lequel la section de préhension (30a) saisit et déploie ledit revers dudit gant, à l'intérieur du module d'enfilage (20), suite au mouvement de la section de préhension (30a), à partir de sa première position d'oscillation, définissant une première extrémité du trajet de mouvement, jusqu'à sa seconde position d'oscillation (30a), définissant une seconde extrémité du trajet de mouvement.

4. Appareil selon l'une quelconque des revendications ci-dessus, comprenant en outre une pédale mobile (40) en prise mobile avec le bras (30) afin qu'un mouvement de la pédale (40) entraîne le mouvement d'oscillation du bras (30).

5. Appareil selon la revendication 4, ladite pédale (40) étant en prise de manière mobile avec la section de manoeuvre (30b) du bras (30).

6. Appareil selon la revendication 4 ou 5, ladite pédale (40) étant en prise de manière mobile avec le bras (30) par l'intermédiaire d'un élément de raccordement (50) pour transmettre (40) le mouvement de la pédale (40) à la section de manoeuvre (30b) du bras (30).

7. Appareil selon la revendication 6, ledit élément de raccordement (50) constituant une barre rigide reliée de manière articulée à la pédale (40) et au bras (30).

8. Appareil selon l'une quelconque des revendications 4 à 6, ladite pédale (40) étant montée de manière oscillante dans l'appareil.

9. Appareil selon l'une quelconque des revendications 4 à 8, comprenant en outre un moyen à ressort (42) destiné à raccorder la pédale (40) au corps de l'appareil pour tendre élastiquement la pédale (40) afin d'entraîner la transition de l'appareil du mode de travail au mode veille après le mouvement de la pédale (40).

10. Appareil selon l'une quelconque des revendications 4 à 9, ladite pédale (40) étant montée au niveau d'une base de l'appareil.

11. Appareil selon la revendication 10, ledit bras (30) étant monté au-dessus de la pédale (40), de préférence de 70 à 120 cm au-dessus de la base de l'appareil.

12. Appareil selon l'une quelconque des revendications ci-dessus, ledit conteneur (11) du module de stockage (10) de gants comprenant une porte (112) permettant d'accéder à l'intérieur du conteneur (11) et de réapprovisionner le conteneur (11) avec les gants.

13. Appareil selon l'une quelconque des revendications ci-dessus, ledit système de pressage (14) de gants comprenant un moyen à ressort (141) terminé par une plaque de pression (142) pour presser de manière élastique les gants dans le conteneur (11) contre la paroi (111) avec l'ouverture (111a).

14. Appareil selon la revendication 13, ladite plaque de pression (142) comprenant un guide (143) destiné à guider la plaque de pression (142), ledit guide (143) reliant de manière articulée la plaque de pression (142) avec la porte (112) du conteneur (11).

15. Appareil selon l'une quelconque des revendications ci-dessus, ledit conteneur (11) du module de stockage (10) de gants comprenant en outre un plateau (144) agencé dans une section inférieure du conteneur (11) pour la collecte de déchets générés après la distribution et l'enfilage de gants.
